# EUROPÄISCHE PATENTSCHRIFT

(11) **EP 0 486 863 B1**
(45) Veröffentlichungstag und Bekanntmachung des Hinweises auf die Patenterteilung: **30.08.1995**
(21) Anmeldenummer: 91118732.6
(22) Anmeldetag: 04.11.1991
(51) Int. Cl.: A61K 31/78, A61K 33/06, A61K 9/20

(54) **Antazida-Zubereitung mit verlängerter Magenverweilzeit**
Antiacid compositions with prolonged gastric residence time
Compositions antiacides à séjour gastrique prolongé

(30) Priorität: 17.11.1990 DE 4036757
(43) Veröffentlichungstag der Anmeldung: 27.05.1992
(73) Patentinhaber: BAYER AG, 51368 Leverkusen (DE)
(72) Erfinder: Fritsch, Christian, Dr., W-8520 Erlangen (DE); Häusler, Franz, Dr., W-5060 Bergisch Gladbach 1 (DE); Seedig, Jürgen, W-5090 Leverkusen 1 (DE)

(56) Entgegenhaltungen:
- WO-A-88/00051
- WO-A-90/07344

## Beschreibung

Vorliegende Erfindung betrifft eine Antazida-Zubereitung mit verlängerter Magenverweilzeit, bestehend aus einer Antazidum-Wirksubstanz, Calcium-Polycarbophil und einem wasserunlöslichen, anionischen Polymer.

Antazida finden breite Anwendung bei der Behandlung verschiedener Magen- und Zwölffingerdarmerkrankungen und -beschwerden wie Gastritis, Reizmagen, Sodbrennen, Reflux, Refluxoesophagitis, Magen- und Zwölffingerdarmgeschwüre sowie nächtlichem Säureschmerz.

Als Antazida finden heute hauptsächlich Aluminiumhydroxide und -carbonate und Magnesiumhydroxide und -carbonate in unterschiedlichem Mischungsverhältnissen Anwendung. Die beiden Hydroxide (bzw. Carbonate) können in Form von physikalischen Mischungen, Trockengelen (z.B. Co Dried-Gels), Naßgelen oder auch sogenannten Schichtgitterantazida miteinander kombiniert sein. Daneben werden - mit geringerer Bedeutung - auch Aluminiumphosphat, Magnesiumtrisilikat und Calciumcarbonat eingesetzt.

Die Wirksamkeit der Antazida beruht primär auf ihrer Fähigkeit, Magensäure zu neutralisieren. Daneben sind aber auch weitere Wirkungsmechanismen, wie die Bindung von Pepsin und Gallensäuren sowie die Stimulierung der Natriumbicarbonat-Sekretion in der Magenschleimhaut durch Aktivierung der Prostaglandin-Synthese bekannt.

Da Antazida ihre therapeutische Wirkung ausschließlich im Inneren des Magens entfalten, ist die Dauer ihrer Wirkung sehr stark von der Entleerungsgeschwindigkeit des Magens abhängig. Es ist lange bekannt, daß die Verweilzeit von Antazida im Magen sehr kurz ist. Literaturzitate schwanken zwischen Angaben von wenigen Minuten bis etwa einer Stunde. Besonders der nüchterne Magen, wie er beispielsweise nachts vorliegt, entleert Antazida recht schnell. Die Kapazität zur Neutralisation von Säure (engl. Acid Neutralization Capacity, ANC) liegt bei handelsüblichen Antazida-Zubereitungen im Bereich von 10 bis 75 mVal pro Einmaldosis. Demgegenüber produzieren Patienten je nach Zeitpunkt, Alter und Krankheitsbild im Bereich von 0 bis 17 mVal Magensäure pro Stunde. Nur bei solchen Personen mit Krankheitsbildern, die mit einer stark gesteigerten Sekretion von Magensäure einhergehen, wie z.B. Ulcus Duodeni-Patienten, werden mehr als 25 mVal pro Stunde gefunden. Aus dieser Vergleichsbetrachtung ergibt sich, daß Antazida in der Regel ihre Funktion, Magensäure zu neutralisieren, immer nur sehr kurz und häufig unvollständig erfüllen können. Oft wird die Säureneutralisationskapazität des Präparates im Magen gar nicht verbraucht. Aufgrund ihrer kurzen Magenpassage sind Antazida, kurz vor dem Zubettgehen gegeben, nicht in der Lage, den nachts auftretenden Säureschmerz zu beeinflussen, da sie bis zu dem Zeitpunkt des Auftretens des Schmerzes bereits aus dem Magen entleert sind.

Dagegen ist eine andere Gruppe von Magentherapeutika, die H2-Antagonisten, befähigt, die Säureproduktion des Magens über einen Zeitraum von mehreren Stunden zu blockieren. Bei diesen Arzneimitteln ist jedoch, nicht zuletzt deswegen, weil sie sich im Unterschied zu den Antazida über das Blut im ganzen Körper verteilen, die Gefahr vergleichsweise gravierender Nebenwirkungen, in Betracht zu ziehen, wie z.B. Veränderungen der Blut- und Leberwerte, Verwirrtheitszustände und die Erhöhung des Prolactin-Spiegels. Daher können H2-Antagonisten die nebenwirkungsarmen Antazida in ihrer breiten Anwendung nicht generell ersetzen.

Es hat in der Vergangenheit nicht an Versuchen gefehlt, die Magenverweilzeit von Antazida zu verlängern.

So wurden beispielsweise verschiedene Gele und Tabletten entwickelt, welche durch ihre Eigenschaft zu schwimmen, länger im Magen verweilen sollen.

Eine solche Schwimmformulierung, enthaltend Natriumhydrogencarbonat, Calciumcarbonat und Natrium-Alginat, wird in der Patentanmeldung GB 1 524 740 beschrieben. Diese Arzneizubereitung erzeugt unter Ausbildung eines Calcium-Alginat Gelgerüstes, welches durch eingeschlossenes Kohlendioxidgas Auftrieb erhält, ein schwimmfähiges Gel (engl. raft), sobald es mit dem Magensaft in Berührung kommt. Es hat sich gezeigt, daß mit dieser Formulierung einige Nachteile verbunden sind. So eignet sich das Prinzip des Schwimmens bei Antazida vor allem zur Behandlung aufsteigender Magensäure (Reflux), nicht jedoch zur Neutralisation der in tieferen Magenabschnitten, wie z.B. dem Antrum, befindlichen Säure. Daher können schwimmende Antacida bei vielen Magen- und Dünndarmerkrankungen nicht optimal wirken. Darüber hinaus versagt der Schwimmeffekt immer dann, wenn der Patient auf der linken Seite zu liegen kommt. Man hat auch gefunden, daS die säurebindenden Eigenschaften aluminium-haltiger Antazida in dem schwimmenden Gelgerüst reduziert sind und die Gerüsteigenschaften unter dem Einfluß von Aluminium verschlechtert sind.

Die Patentanmeldung EP 0 286 085 beschreibt ein verbessertes Schwimmgel, in welchem Natrium-Alginat beispielsweise durch Natrium-Pectinat ausgetauscht ist. Hierdurch lassen sich die zuletzt genannten Nachteile beseitigen. Die eingeschränkte Anwendungsweise (Reflux) und das Versagen des Prinzips bei auf der linken Seite liegenden Patienten bleibt jedoch erhalten.

Es wurden auch Antazida-Tabletten beschrieben, welche durch ihre Eigenschaft, im Magensaft zu schwimmen, eine verlängerte Wirkdauer besitzen sollen. Die Deutsche Offenlegungsschrift 2 611 041 beschreibt eine Zweischichtentablette, bei der eine Schicht retardiert mit Schwimmeigenschaften und die andere Schicht nicht retardiert formuliert wurde. Die retardierte Schicht bildet im Magensaft eine kompakte Gelstruktur aus Methylcellulose aus, welche jedoch nicht befähigt ist, sich über große Teile der Magenwand zu verteilen und somit großflächig Säure zu neutralisieren. Da diese Formulierung nur mit langsamer Geschwindigkeit in einem sehr engen Wirkradius neutralisieren kann, ist sie als Antazidum nicht geeignet.

Ein völlig anderer Weg zur Darstellung eines lang wirkenden Antazidums wird in der PCT-Anmeldung WO 86/06 632 beschrieben. Es wird vorgeschlagen, Antazida in Plätzchen-Gebäck unter Zusatz von Prostaglandin-Vorstufen (z.B. Lecithin) und Ballaststoffen zu inkorporieren. Da eine weitere Vielzahl von Ingredienzien, wie sie bei der Herstellung von Backwaren üblich sind, zugegeben werden und auch ein Backprozeß bei hohen Temperaturen vorgesehen ist, bleibt eine Anwendung dieser Technologie für Arzneimittel ausgeschlossen.

Es sind auch Formulierungen, die Antazida durch die Quell- und Klebeeigenschaften der verwendeten Polymersubstanzen länger im Magen verweilen lassen sollen, beschrieben worden. Bei dieser Vorgehensweise ist zu beachten, daß das Antazidum durch Einschluß in eine gequollene Matrix nicht zu sehr an seiner Reaktion mit Magensäure behindert wird. Die klebenden Eigenschaften der verwendeten Polymere sind daraufhin zu überprüfen, inwieweit sie zu einer guten Haftung speziell auf Schleimhäuten (bioadhesive bzw. mucoadhesive Eigenschaften) führen. Das US-Patent 3 555 151 beschreibt schnell zerfallende Antazida-Tabletten, bestehend aus stark quellenden und klebenden Granulaten, in welchen das Antazidum in Johannisbrotkernmehl eingeschlossen ist. Da im allgemeinen gequollene hochviskose Gebilde keine verlängerte Magenverweilzeit haben, solange sie nicht eine so große Ausdehnung aufweisen, daß sie den ganzen Magen ausfüllen, ist nicht davon auszugehen, daß die beispielsweise vorgeschlagenen Mengen an Quellstoff den erwünschten Effekt erzielen können. In der Erfindung werden die Klebeeigenschaften der Formulierungen an der Wand eines Gefäßes beschrieben, nicht jedoch die an der Magenwand.

Durch quellende bzw. klebende Formulierungen wird auch in der PCT-Anmeldung WO 88/00051 versucht, Antazida mit verlängerter Magenverweilzeit darzustellen. Die verwendeten anionischen Polymere quellen bei pH-Werten über 6 stark an und bilden eine viskose Masse, in der das Antazidum eingeschlossen ist. Bei dieser Erfindung wurde nicht berücksichtigt, daß im optimalen pH-Bereich von 3 bis 5, der durch Gabe von Antazida im Magen erreicht werden soll, der Quelleffekt gar nicht auftritt. Es konnte auch kein Nachweis erbracht werden, daß die gequollenen Polymere, sei es durch ihr viskoses Verhalten alleine oder durch spezifische Klebeeigenschaften auf der Magenwand, in der Lage sind, das Antazidum längere Zeit im Magen zurückzuhalten. Die Reaktionsgeschwindigkeit der Formulierungen mit Salzsäure war stark verlangsamt.

Durch das US-Patent 4 615 697 ist es bekannt geworden, daß spezielle Polymere, wie die wasserunlöslichen, quervernetzten Polyacrylsäuren (Beispiel: Polycarbophil) durch ausgeprägte bioadhesive Eigenschaften befähigt sind, beispielsweise die Magenverweilzeit von Arzneistoffen zu verlängern. Hieraus kann ein verbessertes Resorptionsverhalten für manche Arzneistoffe, wie z.B. Hydrochlorothiazid, resultieren. Die Möglichkeit, durch diese Polymere eine Verlängerung der Magenverweilzeit von Antazida zu erzielen, wurde nicht in Betracht gezogen. Der Grund für dieses Auslassen der Antazida könnte der saure Charakter der verwendeten Polyacrylsäure-Derivate sein, da es hierdurch zu einer Einschränkung der Wirksamkeit von Antazida kommt. Darüberhinaus wurde später durch verschiedene Autoren festgestellt, daß Polycarbophil nicht oder nur ungenügend dazu in der Lage ist, die Magenverweilzeit von Arzneistoffen zu verlängern.

Die genannten Beispiele zeigen, daß es bisher nur in unvollkommener Weise gelungen ist, Antazida-Zubereitungen herzustellen, die folgenden Anforderungen genügen:
- verlängerte Magenverweilzeit, die auch in lebendem Organismus sichergestellt ist;
- Großflächige Verteilung im Magen, d.h. nicht nur lokale Entfaltung der Wirkung;
- Pharmazeutische Formulierung im üblichen Sinne, d.h. Möglichkeit der Anwendung als Arzneimittel
- Nicht wesentlich behinderte, d.h. verlangsamte oder reduzierte, Reaktion mit saurem Magensaft
Die vorliegende Erfindung betrifft Antazida-Zubereitungen mit langer Magenverweilzeit, dadurch gekennzeichnet, daß sie Antazida-Wirkstoffe, die mit dem Calcium-Salz des Polycarbophils unter Verwendung von wasserunlöslichen, anionischen Polymeren als Bindemittel agglomeriert sind, enthalten und dadurch eine hohe Haftfähigkeit (Bioadhesion) auf der Magenschleimhaut und eine verlangsamte Magenpassage besitzen.

Dieser Effekt ist insofern überraschend, da ein verlangsamender Einfluß von Calcium-Polycarbophil auf die Magenpassage nicht zu erwarten war. Es war vielmehr bekannt, daß Calcium-Polycarbophil die Magenverweilzeit von Barium-Zubereitungen, welche vor einer Röntgenuntersuchung eingenommen werden, nicht beeinflußt (Current Therapeutic Research, 6, Seiten 572 bis 583, 1964).

Bekannt ist auch die Anwendung von Calcium-Polycarbophil als Mittel gegen Durchfall und Verstopfung (Antidiarhoikum und Laxans), wegen der quellenden und wasserbindenden Eigenschaften, welche die Substanz im Darm entfaltet. Die vorliegende Erfindung zeigt außerdem unerwarteterweise, daß eine Agglomeration von AntazidaWirkstoffen mit Calcium-Polycarbophil unter Verwendung von solchen wasserunlöslichen anionischen Polymeren, deren übliches Einsatzgebiet die Erzeugung von magensaftresistenten pharmazeutischen Zubereitungen ist, nicht zu einer Verlangsamung der Reaktion des Antazidums mit Salzsäure führt. Üblicherweise werden diese Polymere als magensaftresistente Überzüge eingesetzt, um einen Schutzschild gegen den Angriff von Salzsäure auf den Arzneistoff aufzubauen bzw. den Magen vor freigesetztem Wirkstoff zu schützen.

Die Erfindung betrifft insbesondere Zubereitungen, wie Agglomerate (Granulate, Pellets), welche nach üblichen Methoden wahlweise zu Tabletten (Kautabletten, dispergierbare Tabletten, überzogene Tabletten) gepreßt werden oder in Kapseln oder Sachets gefüllt werden, enthaltend:

| | |
|---|---|
| a) Antazida-Wirksubstanz | 1 Gew.-Teil |
| b) Calcium-Polycarbophil | 0,1-5 Gew.-Teile |
| c) wasserunlösliches, anionisches Polymer | 0,01-1 Gew.-Teile |

und gegebenenfalls noch weitere übliche Hilfs- und Zuschlagstoffe.

Besonders bevorzugt sind Zubereitungen enthaltend:

| | |
|---|---|
| Antazida-Wirksubstanz | 1 Gew.-Teil |
| Calcium-Polycarbophil | 0,5-2 Gew.-Teile |
| wasserunlösliches, anionisches Polymer | 0,1-0,5 Gew.-Teile |

Als Antazida-Wirksubstanz können alle üblichen Wirkstoffe mit säurebindenden bzw. neutralisierenden Eigenschaften eingesetzt werden.

Bevorzugt werden Aluminium- und/oder Magnesium-haltige Antazida eingesetzt. Besonders bevorzugt werden Aluminium-und Magnesium-haltige Antazida mit Schichtgitterstruktur, wie z.B. Hydrotalcit, Magaldrat und Almagat eingesetzt.

Calcium-Polycarbophil ist bekannt [Pharmakopoe der Vereinigten Staaten (USP XXII)] als das Calcium-Salz einer Polyacrylsäure, die mit Divinylglycol quervernetzt ist. Die Substanz wird von der Fa. Goodrich unter den Typenbezeichnungen "Carbopol EX83" und "Carbopol 977" vertrieben. "Carbopol 977" eignet sich aufgrund seiner geringen Teilchengröße (unter 15 »m) besonders gut für den Einsatz in Kautabletten und dispergierbaren Tabletten und wird in den erfindungsgemäßen Agglomeraten bevorzugt eingesetzt.

Als wasserunlösliche, anionische Polymere werden bevorzugt solche eingesetzt, die durch ihre Anwendung als Magensaftresistenz erzeugende Polymere in der Pharmazie bekannt sind, wie z.B.
- Polyacrylsäure-Methacrylat-Copolymer
- Celluloseacetatphtalat
- Hydroxypropylmethylcellulosephtalat
- Celluloseacetatsuccinat
- Celluloseacetatadipinat
- Schellack
- Polyvinylacetatphthalat
- Celluloseacetattrimellitat
- Hydroxypropylmethylcelullose-acetatsuccinat
Besonders bevorzugt wird Polyacrylsäure-Methacrylat-Copolymer eingesetzt. Diese Substanz wird unter der Handelsbezeichnung "Eudragit L 100-55" oder in Form einer 30 %igen wässrigen Dispersion unter dem Namen "Eudragit L 30 D" von der Fa. Röhm, Darmstadt, vertrieben. Sie ist im amerikanischen "National Formulary, NF XVII" unter dem Namen "Methacrylic Acid Copolymer" als "Type C" beschrieben.

Die Herstellung der erfindungsgemäßen Zubereitung erfolgt nach üblichen Methoden wie Agglomeration der drei Komponenten a, b und c unter Einsatz üblicher Agglomerationstechniken, wie der Feuchtgranulation in Mischern oder Wirbelschichtapparaturen, der Trockengranulation, Extrusion, Spheronisation und Pelletisierung.

Die erfindungsgemäßen Antazida-Agglomerate entfalten ihre magenschleimhaut-haftende und schnell neutralisierende Wirkung am besten, wenn sie einen mittleren Korngrößenbereich von 55 bis 2.000 »m haben, Besonders bevorzugt werden mittlere Korngrößen von 100 bis 800 »m.

In den Agglomeraten können auch weitere pharmazeutische Hilfsstoffe wie Füllstoffe, Bindemittel, Weichmacher oder Zerfallshilfsmittel inkorporiert werden. Weiterhin können den Agglomeraten auch pharmazeutisch übliche Zuschlagsstoffe zugemischt werden, wie sie in Tabletten, Kapseln oder Sachets Anwendung finden, Solche Zuschlagsstoffe sind beispielsweise Füllstoffe, Bindemittel, Gleitmittel, Fließregulierungsmittel, Zerfallshilfsmittel (Tablettensprengmittel), Süßstoffe auf Kohlenhydrat-Basis, künstliche Süßungsmittel, Zuckeraustauschstoffe und Aroma-Stoffe.

Werden die erfindungsgemäßen Antazida-haltigen Agglomerate zu Tabletten weiterverarbeitet, so ist darauf zu achten, daß diese durch den Einfluß von Tablettensprengmitteln bei Kontakt mit wäßrigem Milieu leicht zerfallen, um somit eine freie Verteilung der Agglomerate auf der Magenschleimhaut zu ermöglichen. Die Tabletten sollten als Kautabletten oder als leicht in Wasser dispergierbare Tabletten konzipiert sein.

Die vorteilhaften Eigenschaften der erfindungsgemäßen Antazida-Zubereitungen können durch Bestimmung der Antaziden Wirksamkeit (In-Vitro-Methode) und der Klebeeigenschaften an exzidierten Hundemagen-Stücken nachgewiesen werden.

### A) Antazide Wirksamkeit

In einem 300 ml-Becherglas mit Magnetrührer (200 UPM) werden 150 ml 0,1 N Salzsäure bei 37°C temperiert und gerührt. Zur Messung des pH-Wertes wird eine Glaselektrode eingebracht. In die vorgelegte Salzsäure wird eine erfindungsgemäße Tablettenformulierung gegeben (Beispiel 1) und der pH-Wert über einen Schreiber kontinuierlich aufgezeichnet. Nach 10 Minuten und jeweils ständig nach weiteren 10 Minuten werden 10 ml 0,1-N Salzsäure zugetropft. Hierdurch wird die Sekretion des Magens simuliert.

Es wird festgestellt, über welchen Zeitraum das Antazidum in der Lage ist, den pH-Wert des Systems im optimalen pH-Bereich zwischen 3 und 5 zu halten. Weiterhin läßt der Test erkennen, ob das Antazidum schnell das zusätzliche Angebot von 10 ml 0,1-N Salzsäure neutralisiert oder ob ein lang andauernder Abfall des pH-Wertes resultiert.

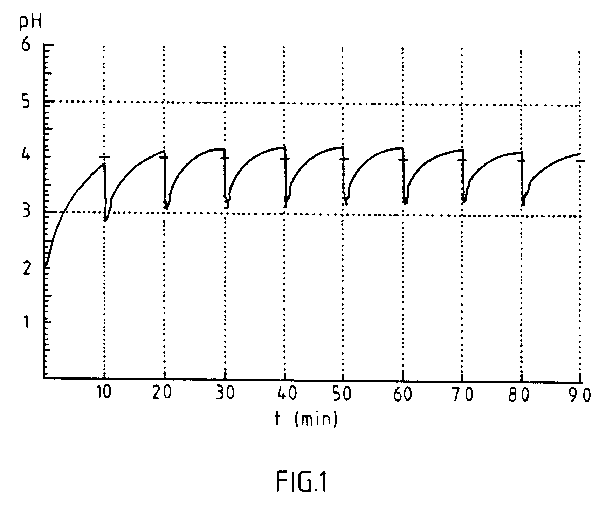

Abb. 1 zeigt das Ergebnis des Antaziden Wirksamkeit-Tests für die Tablettenformulierung nach Beispiel 1.

### B) Untersuchung der Klebeeigenschaften an exzidierten Hundemagenstücken

Es wurde ein einfaches Modell entwickelt, um die Klebeeigenschaften von Antazida-Zubereitungen auf Hundemagenschleimhaut zu bestimmen. Die Magenschleimhaut des Hundes ist mit der des Menschen weitgehend vergleichbar. Dem Magen eines abgetöteten Hundes werden runde Stücke mit einem Durchmesser von etwa 5 cm entnommen. Diese Stücke werden in eine Halterung eingespannt, welche die innere Oberfläche des Magens und damit die Schleimhaut für anschließende Untersuchungen zugänglich macht. Eine wäßrige Aufschlämmung der erfindungsgemäßen Antazida-Zubereitungen wird auf die Oberfläche der Magenschleimhaut gestrichen und die gesamte Halterung in ein Becherglas mit 0,1-N Salzsäure von 37°C eingehängt und intensiv gerührt. Die Haftung der Zubereitung auf der Mucosaoberfläche wird über einen längeren Zeitraum von zum Beispiel 90 min optisch verfolgt.

Während bei Talcid®-Tabletten und anderen handelsüblichen Anticidazubereitungen eine Haftung an der Mucosa nur für maximal 10 Minuten festgestellt werden kann, ist bei der erfindungsgemäßen Zubereitung selbst nach 90 Minuten noch eine starke Anhaftung zu erkennen.

### Beispiel 1

1 g Hydrotalcit enthaltende Tablette mit verlängerter Magenverweilzeit

| | |
|---|---|
| Hydrotalcit | 1.000 mg |
| Polyacrylsäure-Methacrylat-Copolymer (eingesetzt als 30 %ige Eudragit L 30 D®-Dispersion) | 225 mg |
| Polyvinylpyrrolidon 25 | 50 mg |
| 1,2-Propylenglykol | 100 mg |
| Calcium-Polycarbophil | 1.000 mg |
| Crospovidone | 343 mg |
| Hochdisperse Kieselsäure | 50 mg |
| Saccharin-Natrium | 10 mg |
| Bananen-Aroma | 2 mg |
| Calcium-Stearat | 20 mg |

In einem Mischgranulator werden 10 kg Hydrotalcit eingewogen. Es wird weiterhin eine Granulationsflüssigkeit durch Auflösen von 0,5 kg Polyvinylpyrrolidon 25 und 1 kg 1,2-Propylenglykol in 7,5 kg Eudragit L 30 D-Dispersion hergestellt. Die Granulationsflüssigkeit wird dem Hydrotalcit im Mischgranulator unter Rühren zugegeben, wobei ein Feuchtgranulat entsteht. Zu diesem Feuchtgranulat werden 10 kg Calcium-Polycarbophil dazugegeben und intensiv weitergerührt. Das Granulat wird geraspelt, getrocknet und anschließned über ein Sieb der lichten Maschenweite 1 mm gesiebt. Zu dem gesiebten Granulat werden 3,43 kg Crospovidone, 0,5 kg hochdisperse Kieselsäure, 0,1 kg Saccharin-Natrium, 20 g Bananen-Aroma und 0,2 kg Calcium-Stearat zugemischt. Die fertige Mischung wird auf einer Rundlauftablettenpresse zu Tabletten mit einem Gewicht von 2,8 g und einem Durchmesser von 25 mm verpreßt.

Die erhaltenen Tabletten zerfallen in Wasser von 37°C in weniger als zwei Minuten und geben Hydrotalcit-haltige Granulatteilchen mit hoher Klebekraft auf der Magenschleimhaut frei.

### Beispiel 2

800 mg Magaldrat enthaltende Tablette mit verlängerter Magenverweilzeit

| | |
|---|---|
| Magaldrat | 800 mg |
| Celluloseacetatphtalat (eingesetzt als 20 %ige Aquateric®-Dispersion) | 180 mg |
| Polyvinylpyrolidon-Polyvinylacetat-Copolymer | 30 mg |
| Triacetin | 40 mg |
| Calcium-Polycarbophil | 800 mg |
| Natrium-Carboxymethylstärke | 200 mg |
| Hochdisperse Kieselsäure | 30 mg |
| Xylitol | 300 mg |
| Saccharin-Natrium | 5 mg |
| Pfefferminz-Aroma | 5 mg |
| Magnesium-Stearat | 10 mg |

In einem Mischgranulator werden 8 kg Magaldrat eingewogen. Es wird eine Granulationsflüssigkeit durch Auflösen von 0,3 kg Polyvinylpyrrolidon-Polyvinylacetat-Copolymer und 0,4 kg Triacetin in 9 kg Aquateric®-Dispersion hergestellt. Die Granulationsflüssigkeit wird dem Magaldrat im Mischgranulator unter Rühren zugegeben, wobei ein Feuchtgranulat entsteht. Zu diesem Feuchtgranulat werden 8 kg Calcium-Polycarbophil und 1 kg Natrium-Carboxymethylstärke gegeben und intensiv weitergerührt. Das Granulat wird geraspelt, getrocknet und anschließend über ein Sieb der lichten Maschenweite 1 mm gesiebt. Zu dem gesiebten Granulat werden 1 kg Natriumcarboxymethylstärke, 0,3 kg hochdisperse Kieselsäure, 3 kg Xylitol, 50 g Saccharin-Natrium, 50 g Pfefferminzaroma und 0,1 kg Magnesium-Stearat zugemischt. Die fertige Mischung wird auf einer Rundlauftablettenpresse zu Tabletten mit einem Gewicht von 2,4 g und einem Durchmesser von 22 mm verpreßt.

### Beispiel 3

| | |
|---|---|
| Aluminiumhydroxid-Magnesiumhydroxid Co-Dried-Gel | 800 mg |
| Hydroxypropylmethylcellulosephtalat | 150 mg |
| Polyethylenglykol 6000 | 50 mg |
| Crospovidone | 170 mg |
| Calcium-Polycarbophil | 1000 mg |
| Mannitol | 300 mg |
| Saccharin-Natrium | 5 mg |
| Pfefferminz-Aroma | 5 mg |
| Calcium-Stearat | 20 mg |

Die Herstellung erfolgt analog zu den Beispielen 1 und 2. Ein Unterschied besteht darin, daß Hydroxypropylmethylcellulosephtalat nicht aus einer wäßrigen Dispersion heraus, sondern aus einer alkoholischen Lösung verarbeitet wird.

Die erhaltenen Tabletten mit einem Gewicht von 2,5 g und einem Durchmesser von 22 mm zerfallen in Wasser in gut magenschleimhauthaftende Teilchen.

## Patentansprüche (Patentansprüche für folgende(n) Vertragsstaat(en): AT, BE, CH, DE, DK, FR, GB, IT, LI, LU, NL, SE)

1. Antazida-Zubereitungen mit verlängerter Magenverweilzeit, dadurch gekennzeichnet, daß sie Antazida-Wirkstoffe enthalten, die mit dem Calciumsalz des Polycarbophils unter Verwendung von wasserunlöslichen anionischen Polymeren als Bindemittel agglomeriert sind, eine hohe Haftfestigkeit (Bioadhesion) auf der Magenschleimhaut haben und dadurch eine verlangsamte Magenpassage besitzen.

2. Antazida-Zubereitungen gemäß Anspruch 1, dadurch gekennzeichnet, daß sie 1 Gew.-Teil Antazidawirkstoff, 0,1 bis 5 Gew.-Teile Calciumpolycarbophil, 0,01 bis 1 Gew.-Teile wasserunlösliches anionisches Polymer und gegebenenfalls noch weitere übliche Hilfs- und Zuschlagstoffe enthalten.

3. Antazida-Zubereitungen gemäß Ansprüchen 1 und 2 enthaltend 0,1 bis 0,5 Gew.-Teile wasserunlösliches anionisches Polymer.

4. Antazida-Zubereitungen gemäß Ansprüchen 1 bis 3 enthaltend Antazidawirkstoffe die Aluminium und/oder Magnesium enthalten und Schichtgitterstruktur aufweisen.

5. Antazida-Zubereitungen gemäß Ansprüchen 1 bis 4 enthaltend als Wirkstoffe Hydrotalcit, Magaldrat oder Almagat.

6. Antazida-Zubereitungen gemäß Ansprüchen 1 - 5, enthaltend Calcium-Polycarbophil mit einer durchschnittlichen Teilchengröße bis zu 15 »m.

7. Antazida-Zubereitungen gemäß Ansprüchen 1 - 6, enthaltend als wasserunlösliche anionische Polymere eine Verbindung aus der Gruppe Polyacrylsäure-Methacrylat-Copolymer, Celluloseacetatphthalat, Hydroxypropylmethylcellulosephthalat, Celluloseacetatsuccinat, Celluloseacetatadipinat und/oder Schellack.

8. Antazida-Zubereitungen gemäß Ansprüchen 1 bis 7, dadurch gekennzeichnet, daß die Antazida-Agglomerate einen mittleren Korngrößenbereich von 55 bis 2000 »m haben.

9. Verfahren zur Herstellung von Antazida-Zubereitungen gemäß Anspruch 1, dadurch gekennzeichnet, daß man die drei Komponenten a) Antazida-Wirksubstanz, b) Calcium-Polycarbophil und c) wasserunlösliches anionisches Polymer unter Einsatz üblicher Agglomerationstechniken wie der Feuchtgranulation in Mischern oder Wirbelschichtapparaturen, der Trockengranulation, Extrusion, Spheronisation und Pelletisierung agglomeriert und die erhaltenen Agglomerate gegebenenfalls unter Verwendung von üblichen Hilfs- und Trägerstoffen in eine geeignete Applikationsform überführt.

## Patentansprüche (Patentansprüche für folgende(n) Vertragsstaat(en): ES, GR)

1. Verfahren zur Herstellung von Antazida-Zubereitungen mit verlängerter Magenverweilzeit enthaltend Antazida-Wirkstoffe, die mit dem Calciumsalz des Polycarbophils unter Verwendung von wasserunlöslichen anionischen Polymeren als Bindemittel agglomeriert sind, eine hohe Haftfestigkeit (Bioadhesion) auf der Magenschleimhaut haben und dadurch eine verlangsamte Magenpassage besitzen, dadurch gekennzeichnet, daß man die drei Komponenten a) Antazida-Wirksubstanz, b) Calcium-Polycarbophil und c) wasserunlösliches anionisches Polymer unter Einsatz üblicher Agglomerationstechniken wie der Feuchtgranulation in Mischern oder Wirbelschichtapparaturen, der Trockengranulation, Extrusion, Spheronisation und Pelletisierung agglomeriert und die erhaltenen Agglomerate gegebenenfalls unter Verwendung von üblichen Hilfs- und Trägerstoffen in eine geeignete Applikationsform überführt.

2. Verfahren gemäß Anspruch 1, dadurch gekennzeichnet, daß man 1 Gewichtsteil Antazida-Wirkstoff aus der Gruppe Hydrotalzid, Magaldrat oder Almagat, 0,1 bis 5 Gewichtsteile Calciumpolycarbophil mit einer durchschnittlichen Teilchengröße bis zu 15 »m, 0,01 bis 1 Gewichtsteil wasserunlösliches anionisches Polymer und gegebenenfalls weitere übliche Hilfs- und Zuschlagstoffe einsetzt.

## Claims (Claims for the following Contracting State(s): AT, BE, CH, DE, DK, FR, GB, IT, LI, LU, NL, SE)

1. Antacid preparations having a prolonged gastric residence time, characterised in that they contain antacid active compounds which are agglomerated with the calcium salt of polycarbophil using water-insoluble anionic polymers as binders, have a high adhesion (bioadhesion) to the gastric mucosa and as a result have a prolonged gastric passage.

2. Antacid preparations according to Claim 1, characterised in that they contain 1 part by weight of antacid active compound, 0.1 to 5 parts by weight of calcium polycarbophil, 0.01 to 1 part by weight of water-insoluble anionic polymer and, if appropriate, additionally other customary auxiliaries and additives.

3. Antacid preparations according to Claims 1 and 2 containing 0.1 to 0.5 parts by weight of water-insoluble anionic polymer.

4. Antacid preparations according to Claims 1 to 3 containing antacid active compounds which contain aluminium and/or magnesium and have layer lattice structure.

5. Antacid preparations according to Claims 1 to 4 containing hydrotalcite, magaldrate or almagate as active compounds.

6. Antacid preparations according to Claims 1 - 5 containing calcium polycarbophil having an average particle size up to 15 »m.

7. Antacid preparations according to Claims 1 - 6 containing a compound from the group comprising polyacrylic acid/methacrylate copolymer, cellulose acetate phthalate, hydroxypropylmethylcellulose phthalate, cellulose acetate succinate, cellulose acetate adipate and/or shellac as water-insoluble anionic polymers.

8. Antacid preparations according to Claims 1 to 7, characterised in that the antacid agglomerates have a mean particle size range of 55 to 2000 »m.

9. Process for the production of antacid preparations according to Claim 1, characterised in that the three components a) antacid active substance, b) calcium polycarbophil and c) water-insoluble anionic polymer are agglomerated using customary agglomeration techniques such as moist granulation in mixers or fluidised bed equipment, dry granulation, extrusion, formation of spheres and pelletisation and the agglomerates obtained are optionally converted into a suitable administration form using customary auxiliaries and excipients.

## Claims (Claims for the following Contracting State(s): ES, GR)

1. Method of preparing antacid preparations having a prolonged gastric residence time and containing antacid active compounds which are agglomerated with the calcium salt of polycarbophil using water-insoluble anionic polymers as binders have a high adhesion (bioadhesion) to the gastric mucosa and as a result have a prolonged gastric passage, characterised in that the three components a) antacid active substance, b) calcium polycarbophil and c) water-insoluble anionic polymer are agglomerated using customary agglomeration techniques such as moist granulation in mixers or fluidised bed equipment, dry granulation, extrusion, formation of spheres and pelletisation and the agglomerates obtained are optionally converted into a suitable administration form using customary auxiliaries and excipients.

2. Method according to Claim 1, characterised in that 1 part by weight of antacid active compound from the group comprising hydrotalcite, magaldrate or almagate, 0.1 to 5 parts by weight of calcium polycarbophil having an average particle size up to 15 »m, 0.01 to 1 part by weight of water-insoluble anionic polymer and, if appropriate, other customary auxiliaries and additives are employed.

## Revendications (Revendications pour l'(les) Etat(s) contractant(s) suivant(s): AT, BE, CH, DE, DK, FR, GB, IT, LI, LU, NL, SE)

1. Préparations anti-acides à séjour gastrique prolongé, caractérisées en ce qu'elles contiennent des substances actives anti-acides qui sont agglomérées avec le sel de calcium du polycarbophile avec utilisation de polymères anioniques insolubles dans l'eau comme liants, elles ont un grand pouvoir d'adhérence (bioadhérence) à la muqueuse gastrique et elles ont de ce fait un passage ralenti dans l'estomac.

2. Préparations anti-acides suivant la revendication 1, caractérisées en ce qu'elles contiennent une partie en poids de substance active anti-acide, 0,1 à 5 parties en poids de sel de calcium de polycarbophile, 0,01 à 1 partie en poids de polymère anionique insoluble dans l'eau et, le cas échéant, encore d'autres substances auxilaires et additifs.

3. Préparations anti-acides suivant la revendication 1 ou 2, contenant 0,1 à 0,5 partie en poids de polymère anionique insoluble dans l'eau.

4. Préparations anti-acides suivant les revendications 1 à 3, contenant des substances anti-acides qui renferment de l'aluminium et/ou du magnésium et qui présentent une structure en réseau de couches.

5. Préparations anti-acides suivant les revendications 1 à 4, contenant comme substances actives de l'Hydrotalcit, du Magaldrat ou de l'Almagat.

6. Préparations anti-acides suivant les revendications 1 à 5, contenant du sel de calcium de polycarbophile en particules de diamètre moyen allant jusqu'à 15 »m.

7. Préparations anti-acides suivant les revendications 1 à 6, contenant comme polymère anionique insoluble dans l'eau un composé du groupe d'un copolymère acide polyacrylique-méthacrylate, acétate-phtalate de cellulose, phtalate d'hydroxypropylméthylcellulose, acétate-succinate de cellulose, acétate-adipate de cellulose et/ou gomme laque.

8. Préparations anti-acides suivant les revendications 1 à 7, caractérisées en ce que les masses agglomérées d'anti-acides ont un diamètre de grain moyen compris dans la plage de 55 à 2000 »m.

9. Procédé d'obtention de préparations anti-acides suivant la revendication 1, caractérisé en ce qu'on agglomère les trois composants a) substance douée d'activité anti-acide, b) sel de calcium de polycarbophile et c) polymère anionique insoluble dans l'eau en utilisant des techniques classiques d'agglomération telles que granulation par voie humide dans des mélangeurs ou dans des appareils à couche tourbillonnaire, granulation par voie sèche, extrusion, sphéronisation et transformation en pellets et on transforme les masses agglomérées obtenues en une forme d'administration appropriée, en utilisant éventuellement des substances auxiliaires et des supports classiques.

## Revendications (Revendications pour l'(les) Etat(s) contractant(s) suivant(s): ES, GR)

1. Procédé d'obtention de préparations anti-acides à séjour gastrique prolongé contenant des substances douées d'activité anti-acide, qui sont agglomérées avec le sel de calcium du polycarbophile avec utilisation, comme liants, de polymères anioniques insolubles dans l'eau, qui ont une grande adhérence (bioadhérence) à la muqueuse gastrique et dont le passage dans l'estomac est par conséquent ralenti, caractérisé en ce qu'on soumet les trois composants a) substance douée d'activité anti-acide, b) sel de calcium de polycarbophile et c) polymère anionique insoluble dans l'eau en utilisant des techniques classiques d'agglomération telles que granulation par voie humide dans des mélangeurs ou dans des appareils à couche tourbillonnaire, granulation par voie sèche, extrusion, sphéronisation et transformation en pellets et on transforme les masses agglomérées obtenues en une forme d'administration appropriée, en utilisant éventuellement des substances auxiliaires et des supports classiques.

2. Procédé suivant la revendication 1, caractérisé en ce qu'on utilise une partie en poids de substance douée d'activité anti-acide du groupe Hydrotalcit, Magaldrat ou Almagat, 0,1 à 5 parties en poids de sel de calcium de polycarbophile en particules ayant un diamètre moyen allant jusqu'à 15 »m, 0,01 à 1 partie en poids de polymère anionique insoluble dans l'eau et, le cas échéant, d'autres substances auxiliaires et additifs classiques.
